# EUROPEAN PATENT APPLICATION

(11) **EP 4 144 829 A1**
(43) Date of publication of application: **08.03.2023**
(21) Application number: 21797211.6
(22) Date of filing: 18.03.2021
(51) Int. Cl.: C12M 3/00, C12M 1/02, F04B 17/00, F04B 19/00, F04B 17/03

(54) **METHOD FOR CONSTRUCTING SLOW MICROCYCLIC ARTIFICIAL CELL NICHE AND APPARATUS THEREOF**

(30) Priority: 30.04.2020 CN 202010360162; 30.04.2020 CN 202020698481 U
(71) Applicant: Peng, Xingyue, Xiamen, Fujian 361000 (CN)
(72) Inventor: Peng, Xingyue, Xiamen, Fujian 361000 (CN)
(74) Representative: Cabinet Chaillot
(86) International application number: PCT/CN2021/081436
(87) International publication number: WO 2021/218472

(57) **Abstract**

A method for constructing a slow microcyclic artificial cell niche. A cell niche (62) which is isolated from a flow field is provided in the center of the flow field and the cell niche (62) is in communication with the flow field by means of an opening (61), wherein the opening (61) faces a wake (63) formed by means of the flow field flowing around the cell niche (62).

## Description

### TECHNICAL FIELD

The present invention relates to a method and device for constructing an artificial cell nest, and in particular to a method and device for constructing a slow-microcirculation artificial cell nest.

### BACKGROUND

The human body is a three-dimensional fluid system developed from one cell, and the interaction between up to tens of trillions of cells is very complex, including the processes of fluid transportation, molecular diffusion, nanoparticle transportation and the like. The comprehensive simulation of these processes in vitro is important in biomedical research and development of drugs for the treatment of diseases. For example, if cells of various organs or tissues of a human body can be successfully simulated in a lab-on-a-chip, the cells can be used for replacing a real person to carry out drug screening and experiments, humans do not have to suffer pain, and the screening process is greatly accelerated. The most critical technology for the success of simulation is to precisely control the microfluidic environment of cells, because microfluid has tearing force as well as the function of transporting molecules and nanoparticles and the function of exchanging materials. Therefore, as a matter of fact, the precise control of the microfluidic environment means the precise control of the physical and chemical microenvironments. The embryonic stem cell is a cell with the highest requirement on the microenvironments, and must be self-replicated and differentiated in a stem cell nest, and there has been no example of successful culture of embryonic stem cells by the existing microfluidic technology. However, microfluidic-controlled artificial stem cell nests must be achieved in order to perform experiments under precise condition control for drug testing or research. Therefore, we must find a method for constructing an artificial cell nest, and the most effective way to find this method is to use embryonic stem cells as an example to find out the requirement of control of a microenvironment and use the embryonic stem cells to test the technical effectiveness of the artificial cell nest.

The artificial stem cell nest may be designed into a semi-closed space, so that the cells can be protected against strong interference from the outside while exchanging materials with the outside to a certain extent. The embryonic stem cell not only has strict requirements on culture conditions, such as temperature, acidity and culture medium, but also cannot form a clone (i.e. a growing living cell aggregate of a large number of cells) through single cells like tumor cells. It must be a clone consisting of multiple cells at the time of inoculation for culture. This is because the clone consisting of the multiple cells has many cell secretions to form a stem cell microenvironment. If microfluid takes away these secretions, the microenvironment will be destroyed, the cells will die, and the clone will disappear, resulting in the failure of culture. The prior microfluidic technology cannot meet the requirements of control of a stem cell microenvironment in the following aspects: A) no microcirculations, leading to loss of cell secretions; B) flow velocity is too high, flow time is too long or the flow is too frequent, leading to the destruction of a cell clone; and C) no concept of a cell nest, leading to a too high rate of material exchange. Therefore, the construction of a slow microcirculation-controlled cell nest in the human body using internal circulations and external exchanges is a great engineering challenge. So far, there has been no report in literatures on any example of automatic culture of embryonic stem cells in the microfluidic technology.

### SUMMARY

The main purpose of the present invention is to design a method for constructing a slow-microcirculation artificial cell nest and obtain an artificial cell nest capable of realizing microfluid circulation control. Such an artificial cell nest is a convenient cell or tissue microenvironment simulation technology and research method. Stem cell niches can be more accurately established by such a cell nest, and the detailed parameters of the stem cell niches can be set.

In order to achieve the above purpose, the present invention adopts the following technical solution:
Provided is a method for constructing a slow-microcirculation artificial cell nest, wherein a cell nest partially isolated from a flow field is arranged in a center of the flow field, and the cell nest is communicated with the flow field via only one opening facing towards a wake formed by a flow of the flow field flowing alongside the cell nest.

Provided is a method for constructing a flow field in a slow-microcirculation artificial cell nest, wherein the flow field is formed by fluid disposed in the flow field, wherein the fluid is driven to be moved by a mover arranged in the flow field performing periodic motion along a plane of motion; and the mover is driven to move by a driver arranged outside the flow field. Furthermore, the mover is a rotator, and the rotator drives the fluid to rotate and move centrifugally by rotational friction, wherein a negative pressure is generated in a direction of an axis of rotation of the rotator, and a positive pressure is generated in all directions on a plane orthogonal to the axis of rotation of the rotator, thus driving the fluid to move. Furthermore, the mover is a vibrator, and the vibrator generates a positive pressure in a direction of an axis of vibration, and a negative pressure in all directions on a plane orthogonal to the axis of vibration thereof, thus driving the fluid to move.

Furthermore, the vibrator is a spherical magnet with definite N and S poles, and the spherical magnet rolls in the flow field in a reciprocating manner.

Furthermore, the driver consists of a rectangular magnet sheet with definite N and S poles and a driving coil, the rectangular magnet sheet is arranged within the driving coil, the driving coil is connected with an external audio output equipment through an audio cable, and audio input by the audio cable is square wave input.

Furthermore, a method for inputting an audio to the driving coil by the audio cable includes:
Step 1, making the audio into a file in a wav format or a MP3 format containing a left channel and a right channel, a waveform of the audio being square waves;
Step 2, adjusting a frequency of the vibrator by adjusting a frequency of the square waves;
Step 3, copying the file obtained from the audio into an MP3 player;
Step 4, obtaining different sub-audio files according to different frequencies of the left and right channels;
Step 5, editing a playing sequence or setting a loop playback of the sub-audio files in a music playlist of the MP3 player; and
Step 6, playing the music playlist in the MP3 player, and directly outputting audio signals of the sub-audio files to the driving coil through the audio cable.

Provided is a device for constructing a slow-microcirculation artificial cell nest, comprising a body, wherein a fluid is filled in the body, an annular partition wall extends upwards from an inner bottom surface of the body, an upper channel opening and a lower channel opening symmetrical to each other are formed on the partition wall, a vibrator is arranged outside the partition wall at a position corresponding to the upper channel opening and/or the lower channel opening, and the vibrator is driven by an external driver arranged outside the body to reciprocate, thereby forming a flow field; an inner ring is formed in a center of the flow field, an interior of the inner ring is a cell nest, the inner ring is integrated with the body, a diameter of the inner ring is less than that of the partition wall, and an opening facing towards a wake formed by a flow of the flow field flowing alongside the inner ring is formed on the inner ring.

Furthermore, the cell nest is circular or oval.

Furthermore, the cell nest is circular, with a diameter of 10 mm, and a width of the opening is 2 mm.

### Advantageous Effects

According to the present invention, by arranging the cell nest in the microfluidic field and arranging the opening facing towards the wake formed by the flow of the flow field flowing alongside the cell nest, a unique flow field with a plurality of vortexes is formed in the cell nest, so that external materials can rapidly reach deep inside the cell nest through the vortexes, thus finely controlling ultra-slow microcirculations in the cell nest and the rate of exchanging materials between the inside and the outside of the cell nest. By means of such a cell nest, experimental cell microenvironments can be created more accurately, and their detailed parameters can be set more accurately. The operation of the artificial cell nest technology of the present invention is the same as that of conventional culture dishes, and the artificial cell nest technology of the present invention is a quick and convenient cell or tissue microenvironment simulation technology and research method.

The present invention further obtains the simplest mode of driving the fluid to move by means of the vibrator and the rotator according to the symmetry principle, thereby deriving the simplest related structure and driving mode, and therefore the widest range of application scenarios, the lowest manufacturing cost and the longest service life can be achieved. The device is extensible in terms of driving mode, materials, spacial scale, structure, output capacity, etc., the microfluidic field constructed by the device is particularly suitable for establishing a microcirculation system, and the fluid can achieve microcirculation control in the closed space without any interference from the outside; all the recordings of square waves, string music or a symphony orchestra can drive the liquid in the device; and the device is integrated, and can be widely applied in the fields of chemistry, life science, environmental science, medical treatment and public health, etc.

### BRIEF DESCRIPTION OF THE DRAWINGS

FIG. 1 is a schematic structural diagram of a microfluidic culture dish with a cell nest according to the present invention.
FIG. 2 is a schematic top view of the microfluidic culture dish.
FIG. 3 is a schematic diagram of the flow direction of a fluid driven by a mover in the microfluidic culture dish.
FIG. 4 is a schematic diagram of flow directions of four microcirculations formed in the cell nest.

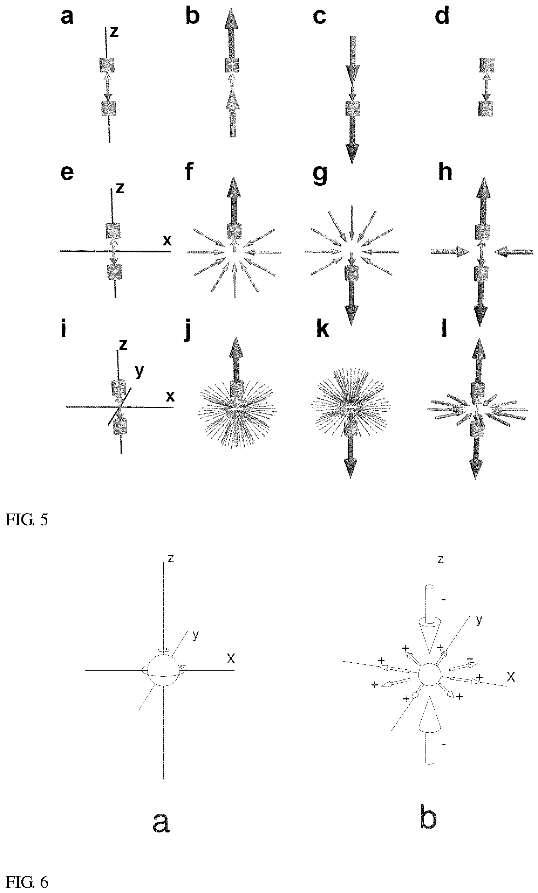

FIG. 5 is a schematic diagram of a vibrator and the influence thereof on the fluid in one-dimensional, two-dimensional and three-dimensional spaces; in which FIGs. 5a-d are schematic diagrams of negative pressures generated by the up-and-down motion of the vibrator along an z-axis in the one-dimensional space; FIGs. 5e-h are schematic diagrams of negative pressures generated by the up-and-down vibration of the vibrator in the two-dimensional space; and FIGs. 5i-l are schematic diagrams of negative pressures generated by the up-and-down vibration of the vibrator along the z-axis in the three-dimensional space.
FIG. 6 is a schematic diagram of the action of a rotator; in which FIG. 6a is a schematic diagram of the rotational direction of the rotator and FIG. 6b is a schematic diagram of a negative pressure generated by the rotation of the rotator.
FIG. 7 is a schematic diagram of one experimental setup according to Embodiment 1 of the present invention.
FIG. 8 is a schematic diagram of actions according to Embodiment 1 of the present invention; in which FIG. 8a is a schematic diagram of an initial state, FIG. 8b is a schematic diagram of the motion of the mover when the magnet sheet rotates clockwise, FIG. 8c is a schematic diagram of the motion of the mover when the magnet sheet rotates counterclockwise, and FIG. 8d is a schematic diagram of the motion of the magnet sheet and the mover under square wave current.

In the accompanying drawings: 1. Body; 2. Partition wall; 21. Upper channel opening; 22. Lower channel opening; 3. Inner annular channel; 4. Outer annular channel; 5. Mover (spherical magnet); 6. Inner ring; 61. Opening; 62. Cell nest; 63. Wake; 7. Rectangular magnet sheet; 8. Driving coil; 9. Cavity wall.

### DETAILED DESCRIPTION

The present invention will be further illustrated in detail with reference to the following embodiments, which, however, are not intended to limit the present invention.

The human body is a fluid system that controls the physical and chemical factors of a microenvironment. Stem cell nests, tumor cell nests, and other semi-enclosed spatial structures involved in tumor cell heterogeneity and stem cell differentiation all need to be simulated for their microfluidic properties in vitro. We are unable to culture embryonic stem cells in existing microfluidic chips because we are unaware of the microfluidic environment of a stem cell nest. In another aspect, the microfluidic chip is far from a simple and easy-to-use technology, and conventional culture dishes lack the microfluidic function.

The present invention relates to a method for constructing a slow-microcirculation artificial cell nest, which can simulate the above microenvironment by arranging, in a microfluidic field, a semi-closed cell nest whose opening is facing towards a wake of a microfluidic flow in the microfluidic field flowing alongside the cell nest to communicate with the microfluidic field. The wake refers to a region where, when there is a fluid flowing against a front side of an object, the pressure of the fluid at a back side of the object is significantly different from that of other parts of the fluid, and this is also called a wake flow. Generally, the wake is presented as a turbulent wake flow of various large and small vortexes at the back side of the object when the fluid separates from the back side of a non-streamlined object after flowing alongside the object.

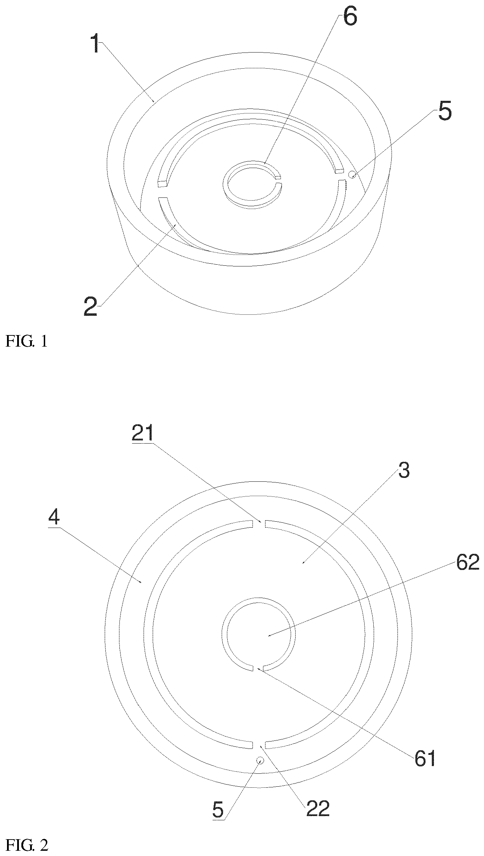

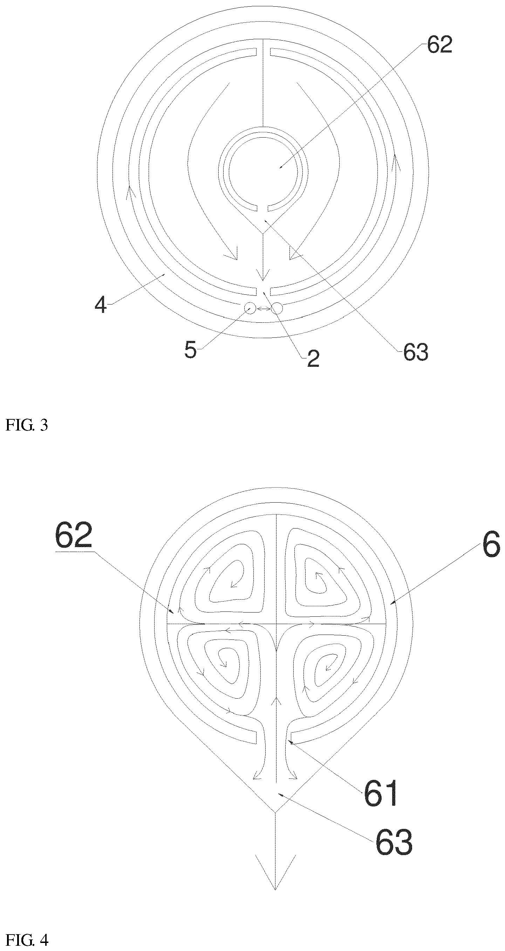

In order to better verify the present invention, the present invention designs a microfluidic culture dish with a cell nest (as shown in FIGs. 1-3) according to the above method. Ultra-slow circulations (< 40 µm/s) and adjustable material exchange rate are achieved to find the optimal conditions for co-culture of tumor cells and stem cells in the artificial cell nest. Specifically, the microfluidic culture dish includes a body 1, and the body 1 is a hollow cylinder with a bottom cover, and a fluid is filled in the body 1. An annular partition wall 2 extends upwards from an inner side of the bottom cover, and the partition wall 2 is integrated with the body 1, so that the body 1 is divided into an inner annular channel 3 and an outer annular channel 4 as two flow fields. An upper channel opening 21 and a lower channel opening 22 are arranged at two symmetrical positions of the partition wall 2 corresponding to a diameter of the partition wall 2. An inner ring 6 with a diameter of 10 mm is further formed in a center of the inner side of the bottom cover of the body 1, the inner ring 6 is also integrated with the body 1, the diameter of the inner ring 6 is less than that of the partition wall 2, and only one opening 61 with a width of 2 mm is formed in the inner ring 6. A central part of the inner ring 6 is a cell nest 62. The opening 61 faces towards a wake 63 formed by the fluid flowing alongside the inner ring 6, preferably facing towards a center of the wake 63. The fluid is filled in the culture dish, and a mover 5 is placed in the outer annular channel 4 corresponding to the lower channel opening 22. The mover 5 is driven by an external driver to move or roll reciprocally in parallel with the inner side of the bottom cover in an area outside the lower channel opening 22, so that the fluid in the culture dish is driven to flow, and thereby a relatively closed cell or tissue culture microenvironment is formed in an area of the cell nest 62 inside the inner ring 6. If the opening 61 of the inner ring 6 is opened towards a different direction, the controllability of microcirculations and the speed of exchange between the inside of the inner ring and the outside of the inner ring are different. If the opening 61 is formed in the wake 63 of the flow field flowing alongside the inner ring, compared with other opening directions of the opening, stable pairs of vortex are formed in the cell nest 62, the vortex pairs have a drawing effect, which not only prevents water from entering but also tapers influent fluid, and thereby, microcirculations are formed in the cell nest 62. As shown in FIG. 4, four circular circulating flow fields in the cell nest 62 can be seen, and there is also a very narrow circulation in a cross-shape circulating throughout the entire cell nest. The four circular circulating flow fields circulate in a clockwise or a counterclockwise direction; the flow fields diagonally opposed to each other circulate in the same direction. When a foreign material fluid was injected into the culture dish, it was found in an experiment that even the highest velocity of cell flow in the cell nest 62 was very low, which is only 2% of the flow velocity outside the cell nest 62. The velocity of a center of each of the vortexes formed in the cell nest and the velocity of a periphery of the cell nest 62 as a whole are even lower. Therefore, a microcirculation area with a low flow velocity is constructed in the cell nest 62, and this circulating flow field is stable, easy to control and particularly suitable for culturing and observing stem cells and the like sensitive to flow velocity.

In order to better implement the control of the above microfluidic field circulation, a method for driving microfluid by employing localized asymmetric motion, as discussed in the Chinese priority application 202010360162.1 (CN111486072A) of the present invention, invented by the same inventor of the present invention, has been introduced into the present invention, in which a mover arranged in a fluid is driven by a driver arranged outside the fluid to perform periodic motion along a plane of motion, thus driving the fluid to move.

The method for driving a microfluid by employing localized asymmetric motion includes vibrator driving and rotator driving. Vibrator driving means that localized vibration of the vibrator generates a positive pressure in a direction of an axis of vibration and a negative pressure in all directions on a plane orthogonal to the axis of vibration thereof, thus driving the fluid to move (as shown in FIG. 5). Rotator driving means that localized rotation of a sphere drives the fluid to rotate and move centrifugally by rotational friction, and a negative pressure is generated in the direction of the axis of rotation, and a positive pressure is generated in all directions on a plane orthogonal to the axis of rotation, thus driving the fluid to move (as shown in FIG. 6).

In the microfluidic culture dish mentioned above, the mover 5 may be driven to move by either one of the two different methods for driving the microfluid as described above.

### Embodiments of the Present Invention

### Embodiment 1:

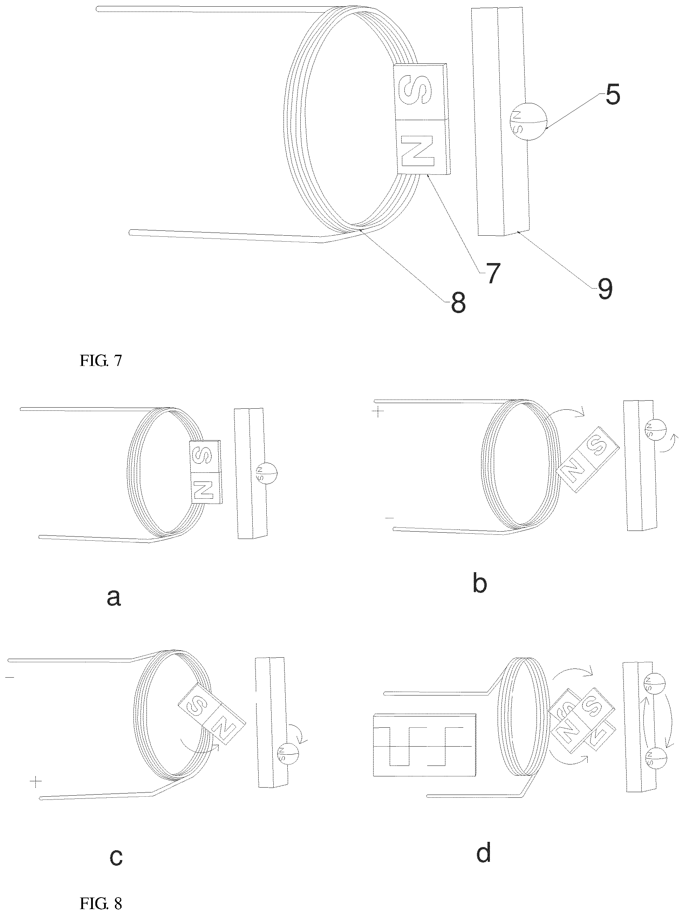

For the vibrator driving mode, the mover 5 may be a spherical magnet with definite N and S poles, and the driver may consist of a rectangular magnet sheet 7 with definite N and S poles and a driving coil 8 (as shown in FIG. 7). The spherical magnet is placed in a fluid cavity, and the rectangular magnet sheet 7 and the driving coil 8 constitute the driver arranged outside the fluid cavity, that is to say, a cavity wall 9 (also called a boundary) is arranged between the driver (rectangular magnet sheet 7 and the driving coil 8) and the mover (spherical magnet). When there is a certain buoyancy, the spherical magnet can be suspended in the fluid. The rectangular magnet sheet 7 is laid within a plane defined by an inner ring of the driving coil 8, and the driving coil 8 is capable of driving the rectangular magnet sheet 7 to rotate or swing. FIG. 7 is a schematic diagram of one experimental setup according to the present embodiment. The spherical magnet, the rectangular magnet sheet 7 and the driving coil 8 are all commercially available. In this experiment, the spherical magnet with a diameter of 2 mm, the rectangular magnet sheet 7 with a length of 4.5 mm and the driving coil 8 with an inner ring width slightly greater than 5 mm and a length of 3 mm are used. The rectangular magnet sheet 7 is placed within the driving coil 8, and the overall size of the whole experimental setup is not greater than 1 cm, so the size is greatly reduced in comparison with that of a prior art microfluid pump. While the same driving principle is maintained, the spherical magnet, the rectangular magnet sheet 7 and the driving coil 8 may be expanded in size according to the requirement of application.

After the driving coil 8 is electrified, the positions of the N pole and S pole of the rectangular magnet sheet 7 are changed by adjusting the current passing through the driving coil 8, so that the position of the spherical magnet is changed. As shown in FIG. 8, when the driving coil 8 is electrified in the forward direction, the S pole of the rectangular magnet sheet 7 swings clockwise towards the spherical magnet with a center of the rectangular magnet sheet 7 as a fulcrum of swing (as shown in FIG. 8b); when the driving coil 8 is electrified in the reverse direction, the N pole swings counterclockwise towards the spherical magnet (as shown in FIG. 8c); when the input current of the driving coil 8 is a square wave current, the rectangular magnet sheet 7 swings reciprocally between the positions indicated in FIG. 8b an FIG. 8c with the center as a fulcrum of swing, and the change of magnetic force causes the spherical magnet on the other side of the cavity wall 9 to roll in a reciprocating manner in a direction parallel to the rectangular magnet sheet 7 (as shown in FIG. 8d). The spherical magnet may slide or roll in the flow field. Because sliding friction force is large, the flow field may be easily blocked or unsmooth, and as a result, the flow field cannot be accurately controlled. In contrast, vibration induced by rolling is standardized. Swinging of the rectangular magnet sheet 7 will produce a sweeping magnetic field that controls the spherical magnet to roll or rotate and thus drives the flow field to produce standard vibration, but the distance and angle of sweeping of the sweeping magnetic field have to correspond to the rolling of the spherical magnet in order to induce ideal vibration driving, i.e., rolling type vibration driving. If not, for example, if the size of the spherical magnet is small, the sweeping magnetic field produced by the swinging of the rectangular magnet sheet 7 to control the spherical magnet will actually shorten the service life of the spherical magnet and its efficiency. In the best correspondence between the rectangular magnet sheet 7 and the spherical magnet is that, each swing of the rectangular magnet sheet 7 causes the spherical magnet to roll for a distance equal to half of its circumference.

Since the size of the spherical magnet is small, the lowest energy required to drive the spherical magnet is only 0.248 mW, meaning that the energy consumption for driving four spherical magnets to vibrate is less than 1 mW. In the present embodiment, sound waves are used as a driving source, the spherical magnet is placed in the outer annular channel 4 corresponding to the lower channel opening 22, and the driving coil 8 (containing the rectangular magnet sheet 7 within) is placed outside the culture dish body 1 close to the spherical magnet, and is connected with an external audio output equipment through an audio cable , that is, two poles of the driving coil 8 are respectively connected to a left channel and a right channel of the external audio output equipment. When stereo audio is played, audio output files are changed into square wave files, so that effective driving can be achieved.

The method for inputting an audio to the driving coil by the audio cable includes:
Step 1, making the audio into a file in a wav format or a MP3 format containing a left channel and a right channel, a waveform of the audio being square waves;
Step 2, adjusting a frequency of the vibrator by adjusting a frequency of the square waves;
Step 3, copying the file obtained from the audio into an MP3 player;
Step 4, obtaining different sub-audio files according to different frequencies of the left and right channels;
Step 5, editing a playing sequence or setting a loop playback of the sub-audio files in a music playlist of the MP3 player; and
Step 6, playing the music playlist in the MP3 player, and directly outputting audio signals of the sub-audio files to the driving coil through the audio cable.

### Embodiment 2:

In the rotator driving mode, the mover 5 is also a spherical magnet with definite N and S poles, and the driver consists of a rectangular magnet sheet with definite N and S poles and a micro-motor (not shown). Similarly, the spherical magnet is placed in the fluid cavity, and the rectangular magnet sheet and the micro-motor as driving bodies are arranged outside the fluid cavity, that is to say, there is a cavity wall between the driver (the rectangular magnet sheet and the micro-motor) and the mover (the spherical magnet). A center of the rectangular magnet sheet is connected with a motor output shaft of the micro-motor, and the rectangular magnet sheet can rotate under the driving of the micro-motor, and the change of magnetic force causes the spherical magnet in the fluid cavity to rotate as well. A length of the rectangular magnet sheet is equivalent to a diameter of the spherical magnet, so that the spherical magnet keeps rotating rather than circular motion. The flow velocity of the flow field is affected by the size of the rotator, and will gradually become weaker with the reduction of the size of the rotator.

By means of the cell nest set in the microfluidic culture dish of Embodiment 1 and Embodiment 2, experimental cell microenvironments can be created more accurately, and their detailed parameters can be set more accurately. For example, when the velocity is < 26 µm/s, running 60 s/day, both stem cells and tumor cells can show good health after they are cultured in the aforementioned artificial cell nest for 14 days. The operation of the artificial cell nest technology according to the present invention is the same as that of conventional culture dish, and the artificial cell nest technology according to the present invention is a quick and convenient cell or tissue microenvironment simulation technology and research method.

The above description is only preferred embodiments of the present invention, and is not intended to limit the technical scope of the present invention, so any minor modifications, equivalent changes and modifications made to the above embodiments according to the technical spirit of the present invention still belong to the protection scope of the present invention.

## Claims

1. A method for constructing a slow-microcirculation artificial cell nest, wherein a cell nest (62) partially isolated from a flow field is arranged in a center of the flow field, and the cell nest (62) is communicated with the flow field via only one opening (61) facing towards a wake (63) formed by a flow of the flow field flowing alongside the cell nest (62).

2. The method for constructing the slow-microcirculation artificial cell nest according to claim 1, wherein the flow field is formed by fluid disposed in the flow field, wherein the fluid is driven to be moved by a mover (5) arranged in the flow field performing periodic motion along a plane of motion; and the mover (5) is driven to move by a driver arranged outside the flow field.

3. The method for constructing the slow-microcirculation artificial cell nest according to claim 2, wherein the mover (5) is a rotator, and the rotator drives the fluid to rotate and move centrifugally by rotational friction, wherein a negative pressure is generated in a direction of an axis of rotation of the rotator, and a positive pressure is generated in all directions on a plane orthogonal to the axis of rotation of the rotator, thus driving the fluid to move.

4. The method for constructing the slow-microcirculation artificial cell nest according to claim 2, wherein the mover (5) is a vibrator, and the vibrator generates a positive pressure in a direction of an axis of vibration, and a negative pressure in all directions on a plane orthogonal to the axis of vibration thereof, thus driving the fluid to move.

5. The method for constructing the slow-microcirculation artificial cell nest according to claim 4, wherein the vibrator is a spherical magnet with definite N and S poles, and the spherical magnet rolls in the flow field in a reciprocating manner.

6. The method for constructing the slow-microcirculation artificial cell nest according to claim 5, wherein the driver consists of a rectangular magnet sheet (7) with definite N and S poles and a driving coil (8), the rectangular magnet sheet (7) is arranged within the driving coil (8), the driving coil (8) is connected with an external audio output equipment through an audio cable, and audio input by the audio cable is square wave input.

7. The method for constructing the slow-microcirculation artificial cell nest according to claim 6, wherein a method for inputting an audio to the driving coil (8) by the audio cable includes:
Step 1, making the audio into a file in a wav format or a MP3 format containing a left channel and a right channel, a waveform of the audio being square waves;
Step 2, adjusting a frequency of the vibrator by adjusting a frequency of the square waves;
Step 3, copying the file obtained from the audio into an MP3 player;
Step 4, obtaining different sub-audio files according to different frequencies of the left and right channels;
Step 5, editing a playing sequence or setting a loop playback of the sub-audio files in a music playlist of the MP3 player; and
Step 6, playing the music playlist in the MP3 player, and directly outputting audio signals of the sub-audio files to the driving coil (8) through the audio cable.

8. A device for constructing a slow-microcirculation artificial cell nest of claim 1, comprising a body (1), wherein a fluid is filled in the body (1), an annular partition wall (2) extends upwards from an inner bottom surface of the body (1), an upper channel opening (21) and a lower channel opening (22) symmetrical to each other are formed on the partition wall (2), a vibrator is arranged outside the partition wall (2) at a position corresponding to the upper channel opening (21) and/or the lower channel opening (22), and the vibrator is driven by an external driver arranged outside the body (1) to reciprocate, thereby forming a flow field; an inner ring (6) is formed in a center of the flow field, an interior of the inner ring (6) is a cell nest (62), the inner ring (6) is integrated with the body (1), a diameter of the inner ring (6) is less than that of the partition wall (2), and an opening (61) facing towards a wake (63) formed by a flow of the flow field flowing alongside the inner ring (6) is formed on the inner ring (6).

9. The device for constructing a slow-microcirculation artificial cell nest according to claim 8, wherein the cell nest (62) is circular or oval.

10. The method for constructing a slow-microcirculation artificial cell nest according to claim 9, wherein the cell nest (62) is circular, with a diameter of 10 mm, and a width of the opening (61) is 2 mm.
